# EUROPEAN PATENT SPECIFICATION

(11) **EP 2 068 721 B1**
(45) Date of publication and mention of the grant of the patent: **29.06.2011**
(21) Application number: 07837338.8
(22) Date of filing: 24.08.2007
(51) Int. Cl.: A61B 17/00, A61B 17/12

(54) **OCCLUDING DEVICE**
VERSTOPFUNGSVORRICHTUNG
APPAREIL D'OCCLUSION

(30) Priority: 24.08.2006 US 509135
(43) Date of publication of application: 17.06.2009
(73) Proprietor: Boston Scientific Limited, Christ Church (BB)
(72) Inventor: EIDENSCHINK, Tracee, E.J., Wayzata, MN 55391 (US); JAGGER, Karl, A., Deephaven, MN 55331 (US); TOMASCHKO, Daniel, Savage, MN 55378 (US)
(74) Representative: Vossius & Partner
(86) International application number: PCT/US2007/018781
(87) International publication number: WO 2008/024491

(56) References cited:
- EP-A- 1 157 663
- WO-A-95/27448
- US-A- 5 108 420

## Description

### FIELD OF THE DISCLOSURE

The present disclosure relates generally to devices for use with cardiac defects; and more particularly to devices for occluding at least a portion of a lumen in a cardiac system.

### BACKGROUND

During development of a fetus in utero, blood flow bypasses the lungs by entering the right atrium and crossing the foramen ovale into the left atrium of the heart. At birth, right heart pressure and pulmonary vascular resistance drop as pulmonary arterioles open in reaction to oxygen filling the alveolus. Left atrial pressure may also rise as the amount of blood returning from the lungs increases. Either or both of these mechanisms may cause the closure of the foramen ovale. The fusion is generally complete by age two in about seventy-five (75) percent of the population, however, patency remains in the other twenty-five (25) percent, resulting in a patent foramen ovale. A patent foramen ovale (PFO) is a residual, oblique, slit-shaped defect resembling a tunnel. Similar defects may occur in other regions within a septum between chambers of the heart, such as atrial septal defects, ventricular septal defects, and the like.

To close such defects, open surgery may be performed to ligate and close the defect. Such procedures are highly invasive and pose substantial morbidity and mortality risks.

Alternatively, catheter based procedures have been developed involving introducing umbrella-like structures into the heart that include opposing expandable structures connected by a hub. One of the expandable structures is inserted through the defect, and both are expanded to secure the tissue surrounding the defect between the structures in an attempt to seal and close the defect. Such structures, however, involve frame structures that support membranes, both of which may fail during the life of the patient being treated, opening the defect, and/or releasing segments of the structure within the patient's heart.

Examples of other structures include those found in WO 95/27448 and EP 1157663.

WO95/27448 disposes a vascular or cardiac filter comprising a self expanding structure made from two conical meshes joined at their apices to form an hourglass profile. The proximal and distal ends of the device having a ring member from which anchoring member extend to fix the device in place.

Accordingly, apparatus for closing patent foramen ovale, patent ductus arteriosus, or other septal defects would be useful.

### BRIEF DESCRIPTION OF THE DRAWINGS

Figure 1 illustrates an embodiment of a device according to the present disclosure.
Figure 2 illustrates an embodiment of a device according to the present disclosure.
Figure 3A-3B illustrate an embodiment of a device according to the present disclosure.
Figure 4 illustrates an embodiment of a delivery device according to the present disclosure.
Figure 5A illustrates an embodiment of a device according to the present disclosure.
Figure 5B illustrates the device illustrated in Figure 5A when a torque is applied according to the present disclosure.
Figure 5C illustrates the device illustrated in Figure 5A when the device is compressed according to the present disclosure.
Figure 6 illustrates an embodiment of a delivery device according to the present disclosure.
Figure 7 illustrates an embodiment of a delivery device according to the present disclosure.

### DETAILED DESCRIPTION

Embodiments of the present disclosure are directed to devices for occluding cardiac defects. A "cardiac defect" can include, but is not limited to, a tunnel-like opening, or body passage, caused by the defective cardiac formation of biological material, where blood can flow through the opening. Examples of cardiac defects include, but are not limited to, patent foramen ovale, patent ductus arteriosus, atrial septal defects, or ventricular septal defects. As used herein, a "defect" as used in "defective cardiac formation" can include an imperfection, malformation, dysfunction, or absence of biological material. For example, an atrial septal defect is a congenital or idiopathic defect in the septum between the atria of the heart, due to failure of the foramen primum or secundum to close and/or form normally. On the other hand, a ventricular septal defect is a congenital defect in the septum between the cardiac ventricles, usually resulting from failure of the spiral septum to close the interventricular foramen. In addition, a patent foramen ovale is an incomplete fibrous adhesion of an adequate valvula foraminis ovalis in the postnatal closure of the foramen ovale. As used herein, "biological material" and/or "tissue" can include, but is not limited to, biological tissue in the body including cardiac muscle tissue, interstitial tissue, smooth muscle tissue, connective tissue, endocardium tissue, tissue formed by apoptosis, or septum tissue.

According to the present disclosure, there are several applications that may benefit from the devices as discussed herein. Such applications include the occlusion of cardiac defects such as a patent foramen ovale. In addition, embodiments of the present disclosure may be useful to correct other atrial septal defects, or ventricular defects such as a ventricular septal defect. Other applications are also possible. In some cases, instead of one cardiac defect, there are several small openings or tunnels that extend from the right atrium or right ventricle through the septum to the left atrium or ventricle, respectively. In these cases, more than one device of the present disclosure can be used to occlude the multiple defects.

Embodiments of the present disclosure provide for a frame defining a lumen, where the frame expands from a first configuration to a second configuration larger than the first configuration. In addition, an anchoring member extends from the frame to secure the frame in the second configuration, and a plug portion of the frame occludes at least a portion of the lumen. As used herein, a "lumen" is defined as the inner open space or cavity defined by the frame of the device. As used herein, "plug portion" refers to a section of the frame either forming a part of the frame and/or attached to a portion of the frame that is used to occlude at least a portion of the lumen. As used herein, "occlude" refers to causing a body passage to become closed or at least partially closed, or preventing the passage of blood and/or particles in the blood through a body passage.

Two plug portions are described by way of example and not by way of limitation. In one embodiment the plug portion can be a filter that allows some fluid through the lumen but prevents blood clots and/or other large particles from passing through the lumen. In this embodiment, the plug portion acts to restrict flow through the lumen enough to allow cell growth in the lumen. By slowing down the flow of blood through the lumen, endothelialization can occur on the frame and the plug portion to close the lumen. In some embodiments, the plug portion can be an impermeable solid that prevents all flow through the lumen.

As used herein, "anchoring member" can include a part of the frame that prevents migration or movement of the frame. In one embodiment, the anchoring member can extend outward beyond a prevailing external line or surface of the frame. In an additional embodiment the anchoring member can be a part of the frame that flares at both ends of the cardiac defect to hold the frame in place inside of the cardiac defect.

In some embodiments, the anchoring member may be in the form of a hook, a shaft, or a barb. As the frame is expanded from the first configuration to the second configuration, the anchoring member can secure the frame to the interior wall of a cavity, e.g., cardiac defect, as will be more fully discussed herein.

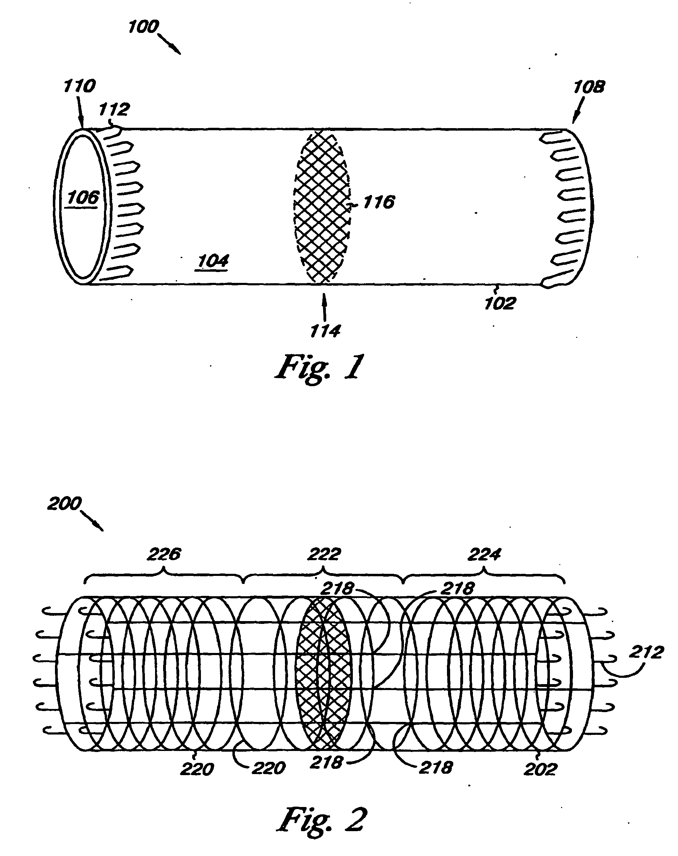

The figures herein follow a numbering convention in which the first digit or digits correspond to the drawing figure number and the remaining digits identify an element or component in the drawing. Similar elements or components between different figures may be identified by the use of similar digits. For example, 110 may reference element "10" in Fig. 1, and a similar element may be referenced as 210 in Figure 2. As will be appreciated, elements shown in the various embodiments herein can be added, exchanged, and/or eliminated so as to provide a number of additional embodiments of value. In addition, discussion of features and/or attributes for an element with respect to one Figure can also apply to the element shown in one or more additional Figs. Also, the figures herein are not necessarily to scale.

Figure 1 provides an illustration of a device 100 according to the present disclosure. As illustrated, the device 100 includes a frame 102 having a first surface 104 and a second surface 106 opposite the first surface 104.

Figure 1 illustrates the frame 102 having a uniform cylindrical shape with a circular cross section. The frame 102 is not limited to this shape, however, and can have cross sections that are elliptical, flattened circular, rectangular, or the like. In addition, in some embodiments, the frame 102 in the second configuration can have a variety of cross-sectional shapes along the length of the frame 102 depending on the shape of the cardiac defect instead of having a uniform shape.

Also, the frame 102 shape and expansion size can be physiologically based depending on the physical aspects of the body passage in which the frame 102 is to be implanted. For example, patent foramen ovale (PFO) can have a large range of dimensions, with a length ranging from five (5) to sixteen (16) millimeters (mm) and a diameter ranging from four (4) to fourteen (14) mm. The device 100 can be configured to fit a particular body passage, therefore, by adjusting the size of frame 102 in the second configuration, the length, and/or the width of the frame 102.

In one embodiment, the frame 102 can have a length between the proximal end 108 and distal end 110 such that the device 100 can extend from one end of the body passage to the other end of the body passage. In another embodiment, the frame 102 can have a length between the proximal end 108 and the distal end 108 that is shorter than the body passage. Other embodiments of the frame 102 are discussed herein.

The embodiments of the frame 102 can be formed from one or more contiguous frame members. The single contiguous member can be bent around an elongate tubular mandrel to form the frame 102. The free ends of the single contiguous member can then be welded, fused, crimped, or otherwise joined together to form the frame 102. In an additional embodiment, the frame 102 can be derived (e.g., laser cut, water cut) from a single tubular segment. The frame 102 can be heat set by a method as is typically known for the material which forms the frame 102.

The embodiments of the frame 102 described herein can be constructed of one or more of a number of materials including metals, polymers, or composites, and in a variety of configurations. In addition, in one embodiment, the frame 102 can be self-expanding. Examples of self-expanding frame 102 materials include temperature-sensitive shape memory polymers (SMPs) or temperature-sensitive shape memory alloys which change shape at a designated temperature or temperature range, as discussed herein. Alternatively, the self-expanding frames 102 can include those having a spring bias. For example, the frame 102 can be formed from an elastic material that can be deformed and then recover its shape after the deformation force is removed. In addition, the frame 102 can have a configuration that allows the frame 102 embodiments be radially expandable through the use of a balloon catheter.

In one embodiment, the frame 102 can be made of a SMP. A SMP is a polymer that has an elasticity modulus which shows a reversible change with a glass transition temperature as the border. When heated above the glass transition temperature (T_{g}), the shape of the SMP can be changed, and the SMP will retain a memory of that shape when cooled below the glass transition temperature. When heated up again above the glass transition temperature, the SMP exhibits a shape recover characteristic by autonomously returning to the original shape. A SMP made from polyurethanes made from polyols and isocyanates has a glass transition temperature freely adjustable between negative forty (-40) and one hundred twenty (120) degrees Celsius by controlling the type of material component (molecular structure), molecular weight, and composition. In the present disclosure, T_{g} for a suitable SMP can be in a range of 40°C to 80 °C.

Some embodiments of the present disclosure can use a frame 102 made of a shape memory alloy. A shape memory alloy works similarly to a SMP, however, the material is comprised of metal alloys instead of a polymer matrix. A specific example of a shape memory alloy that undergoes a change at a glass transition temperature is a nickel-titanium alloy. In such embodiments, the frame 102 can be formed from a nickel-titanium alloy film, foil, or sheet. Other examples of memory metal alloys include titanium-palladuim-nickel, nickel-titanium-copper, gold-cadmium, iron-zinc-copper-aluminum, titanium-niobium-aluminum, hafnium-titanium-nickel, iron-maganese-silicon, nickel-titanium, nickel-iron-zinc-aluminum, copper-aluminum-iron, titanium-niobium, zirconium-copper-zinc, and nickel-zirconium-titanium. Use of other shape memory alloys are also possible.
A frame 102 can be made by forming the frame 102 from a SMP and/or shape memory alloy. In this embodiment, when heat is applied through a conductive wire above the glass transition temperature, the frame 102 can expand from the first configuration to the second "remembered" configuration. On the other hand, the shape memory material can be coated onto a frame 102 constructed from a different material. In this embodiment, when the shape memory coating is heated, the coating could expand the frame 102 from the first configuration to the second configuration.

In one embodiment, as discussed herein, the frame 102 can be made of an elastic material that can be deformed and then recover its shape after a deformation force is removed. For example, in one embodiment the frame 102 can be made of a metal and/or metal alloy. Examples of such metals/metal alloys include, but are not limited to, platinum, cobalt, chromium, titanium, stainless steel (e.g., 316L stainless steel), and gold. In some embodiments, the frame 102 can be made of a plastically deformable polymer, such as expanded polytetrafluoroethylene (ePTFE). Use of other plastically deformable materials is also possible.

In an alternative embodiment, the frame 102 can be made of a biocompatible material that will slowly degrade in the body. In such embodiments, the frame 102 can have a variable thickness where the frame 102 is thickest towards the middle of the device 100, and most thin at the ends of the device 100. Examples of biodegradable materials include, but are not limited to, polycarboxylic acid, polylactic acid, polyhydroxybuterate, polyanhydrides including maleic anhydride polymers; polyorthoesters; poly-amino acids; polyethylene oxide; polyphosphazenes; polyactic acid, polyglycolic acid and copolymers and copolymers and mixtures thereof such as poly(L-lactic acid) (PLLA), poly (D,L,-lactide), poly(lactic acid-co-glycolic acid), 50/50 (DL-lactide-co-glycolide); polydioxanone; polypropylene fumarate; polydepsipeptides; polycaprolactone and co-polymers and mixtures thereof such as poly(D,L-lactide-co-caprolactone) and polycaprolactone co-butylacrylate; polyhydroxybutyrate valerate and blends; polycarbonates such as tyrosine-derived polycarbonates and arylates, polyiminocaronates, and polydimethyl-trimethylcarbonates; cyanoacrylate; calcium phosphates; polyglycosaminoglycans; macromolecules such as polysaccharides (including hyaluronic acid, cellulose, and hydroxypropylmethyl cellulose; gelatin; starches; dextrans; alginates and derivatives thereof), proteins and polypeptides; and mixtures and copolymers of any of the foregoing.

In further embodiments, the frame 102 can include one or more therapeutic agents. In one embodiment, the one or more therapeutic agents can be integrated into the frame 102 material matrix and/or coated on either the first surface 104 and/or second surface 106. The one or more therapeutic agents can then leach and/or be released from the frame 102 once it is implanted. Examples of therapeutic agents include, but are not limited to, pharmaceutically acceptable agents such as non-genetic therapeutic agents, a biomolecule, a small molecule, or cells. The therapeutic agents may be combined to the extent such combination is biologically compatible.

In addition, the frame 102 material may be used in conjunction with radiopaque filler materials such as barium sulfate, bismuth trioxide, bismuth carbonate, powdered tungsten, powdered tantalum, or the like so that the location of the device 100 may be radiographically visualized within the human body.

The device 100 can further include anchoring members 112 associated with the first surface 104. As discussed herein, the anchoring members 112 extend from the frame 102 and can be used to secure the frame 102 to the interior of a body passage. The anchoring members 112 can be in the form of a barb, a hook, a ring, a flare, or a shaft. The anchoring members 112 can engage the interior of a body passage when the frame 102 is expanded from a first configuration to a second configuration larger than the first configuration, securing the frame 102 to the interior of the body passage. In some embodiments, the anchoring members 112 can be provided at the ends of the frame 102, as shown in Figure 1. In various embodiments, the anchoring members 112 can be provided along the entire frame 102 and/or in a middle portion of the frame 102. The anchoring members 112 can also extend from the frame 102 in other places.

In some embodiments, the second surface 106 can provide the anchoring members 108. In such embodiments, the anchoring members 112 can extend from the second surface 106 through the frame 102, so that the anchoring members 112 engage the tissue defining the body passage similarly to when the anchoring members 112 extend from the first surface 104, as discussed herein. On the other hand, the anchoring members 112 can extend from the second surface 106 at the ends of the frame 102 such that they extend in a direction approximately parallel to the second surface beyond the ends of the frame 102. The anchoring members 112 in this embodiment can be in the form of a hook, where the hook-portion of the anchoring members 112 is approximately perpendicular to the second surface 106. The anchoring members 112 can engage the tissue defining the interior of the body passage when the frame 102 is expanded from the first configuration to the second configuration, as discussed herein.

The anchoring members 112, either on the first surface 104 or the second surface 106, can be integrally formed from the frame 102 in such a way that allows the anchoring members 112 to be folded, or bent, to an upright position relative the surface of the remaining portion of the frame 102. In such embodiments, the anchoring members 112 can be integrally formed from the frame 102 by laser-cutting, etching, or stamping, or the like, and then plastically deformed outward. Additionally, the anchoring members 112, either on the first surface 104 or the second surface 106, can be integrally formed from the frame 102 in such a way that allows the anchoring members 112 to project away from the surface of the remaining portion of the frame 102 when the frame is expanded from the first configuration to the second configuration.

In various embodiments of the present disclosure, the anchoring members 112 can be formed of a different material than the frame 102, or of the same material as the frame 102, and the anchoring members 112 are joined to the frame 102. In such embodiments, the anchoring members 112 can be joined to the frame 102 using a chemical adhesive, or by laser welding, among other techniques.

To engage the interior of a body passage, the frame 102 is configured so that the perimeter of the frame 102 in the second configuration is at least as large as the original circumference of the body passage. Then, when the frame 102 expands from the first configuration to the second configuration, the anchoring members 112 can engage the tissue of the body passage as the frame 102 expands. Once the anchoring members 112 engage and anchor into the tissue defining the body passage, the anchoring members 112 can help to hold the frame 102 in place inside the body passage, preventing migration of the device 100.

Figure 1 also illustrates a plug portion 114 associated with the frame 102. As discussed herein, the plug portion 114 can have a number of different configurations that are used to filter and/or occlude the flow of blood through the device 100. For example, a filter 116 can be used as the plug portion 114 where the filter 116 is coupled to the perimeter of the second surface 106 and/or the first surface 104 to form the plug portion 114. The filter 116 can be coupled to the perimeter of the second surface 106 and/or the first surface 104 using staples, adhesives, sutures, chemical reaction, laser welding, or the like.

In addition, although the plug portion 114 is shown in the middle of the frame 102, in various embodiments the plug portion 114 can be placed closer to one end of the frame 102, in the middle, or at an end of the frame 102.

In a filter embodiment, the filter 116 can be constructed of a porous biocompatible material that can be either synthetic or biologic. The filter 116 can be a woven material or a perforated material. In one embodiment, the plug portion 114 can be formed from a fluid-impermeable biocompatible material that can be either synthetic or biologic, and prevents the flow of blood through the lumen, as discussed herein.

Possible synthetic materials include, but are not limited to, expanded polytetrafluoroethylene (ePTFE), polytetrafluoroethylene (PTFE), polystyrene-polyisobutylene-polystyrene, polyurethane, segmented poly(carbonate-urethane), dacron, polyethlylene (PE), polyethylene terephthalate (PET), nafion carbon nanotubes, silk, urethane, rayon, silicone, or the like.

Possible biologic materials include, but are not limited to allogeneic or xenograft material. These include explanted veins and decellularized basement membrane materials, such as small intestine submucosa (SIS) or umbilical vein. Additional biologic materials include, but are not limited to, peptides, polypeptides and proteins; oligonucleotides; nucleic acids such as double or single stranded DNA (including naked and cDNA), RNA, antisense nucleic acids such as antisense DNA and RNA, small interfering RNA (siRNA), and riobozymes; genes; carbohydrates; angiogenic factors including growth factors; cell cycle inhibitors; and anti-restenosis agents.

Figure 2 is an illustration of some embodiments of a device 200 according to the present disclosure. As illustrated the device 200 includes a frame 202 where the frame 202 can be more flexible in some portions of the frame 202 as compared with other portions of the frame 202 depending on how many junction points 218 are provided in that portion of the frame 202. As used herein, a "junction point" 218 is where the frame members 220 intersect and join.

In such embodiments, the device 200 has a middle portion 222 that is more flexible than the first end portion 224 and second end portion 226 of the frame 202 because the middle portion 222 has less junction points 218 than the first and second end portions 224, 226.

In one embodiment, the device 200 can be configured so that the first and second end portions 224, 226 are more flexible than the middle portion 222 of the frame 202 by having more junction points 218 in the middle portion 222 as compared to the number and/or location of junction points 218 at the end portions 224, 226.

In an additional embodiment, the middle portion 222 and either the first end portion 224 or the second end portion 226 can be more rigid than the other end portion by adding junction points 218 to the middle portion 222 and one end portion of the frame 202.

In one embodiment, the frame 202 flexibility can be varied by using a flexible material in the middle portion 222 of the frame 202 and a rigid material at the end portions 224, 226 of the frame 202. In this embodiment, the materials can be connected to form the frame 202 by laser welding, chemical adhesion, or the like.
In yet another embodiment, the frame 202 flexibility can be varied by adjusting the thickness of the members 220 forming the frame 202. Where the frame 202 is desired to be rigid, the frame members 220 can be thicker than where the frame 202 is desired to be flexible.

In one embodiment, the frame 202 flexibility can be varied by varying the cross-sectional shapes of the frame members 220. For example, the frame members 220 can have cross sectional shapes such as circular, polygonal, oval, I-shaped, and/or T-shaped. Other cross-sectional shapes are also possible. In this embodiment, the members 220 forming the frame 202 of differing shapes can be connected to form the frame 202 by laser welding, chemical adhesion, or the like.

In one embodiment, the anchoring members 212 can be positioned on the ends 224, 226 of the frame 202, where the frame 202 is the most rigid because of the additional junction points 218. For example, the anchoring members 212 can be placed wherever the frame 202 is most rigid so that when the frame 202 expands, the anchoring members 212 can be firmly secured to the interior of the body passage. As another example, if the middle section 222 of the frame 202 is configured to be rigid by the addition of junction points 218, then anchoring members 212 can extend from the middle section 222 of the frame 202.

In some embodiments, the anchoring members 212 could be secured to the interior of the body passage using a delivery device, such as a catheter equipped with an inflatable balloon, as discussed herein. In these embodiments, the anchoring members 212 can be placed on the frame 202 regardless of where the frame 202 is rigid. By providing an inflatable balloon, the frame 202 will be held rigid while the inflatable balloon is inflated, even in areas of greater flexibility, which will allow the anchoring members 212 to engage with the tissue of the body passage.

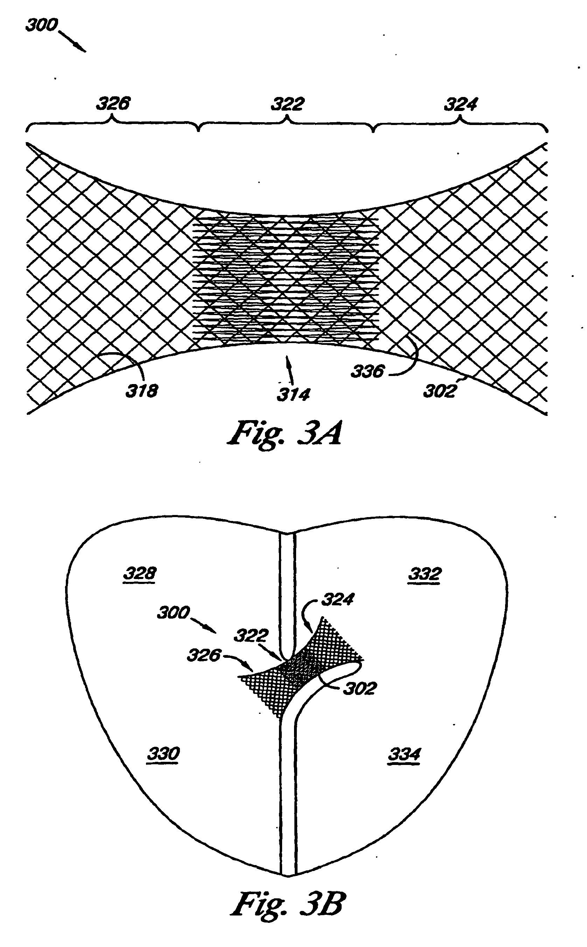

Figures 3A-3B illustrate an embodiment of the device 300 where the device 300 has a frame 302 with a first end portion 324, a second end portion 326, and a middle portion 322 when the frame 302 has been expanded from a first configuration to a second configuration larger than the first configuration. In this embodiment the frame 302 expands from a first configuration to a second configuration, however, in addition, the first and second end portions 324, 326 in the second configuration have a larger perimeter than the middle portion 322 in the second configuration. The frame 302 can expand using a balloon catheter and/or using self-expanding materials, as discussed herein. As shown in Figure 3B, in this embodiment, the first and second end portions 324, 326 can anchor the frame 302 to the body passage, where the device 300 extends between the right atrium 328 or right ventricle 330 via a lumen to the left atrium 332 or left ventricle 334, respectively.

In one embodiment, the frame 302 can have areas of greater flexibility by having fewer junction points 318 in a portion of the frame 302 that is desired to be flexible as compared to a portion of the frame 302 that is desired to be rigid, as discussed herein.

In some embodiments, the middle portion 322 of the frame 302 can have anchoring members, as discussed herein. The additional anchoring members on the middle portion 322 of the frame can act with the first end portion 324 and second end portion 326 to hold the frame 302 inside the body passage and prevent migration of the device 300.

In one embodiment, a plug portion 314 can be attached to the frame 302 to filter the flow of blood through the lumen, or to prevent the flow of blood through the lumen, as discussed herein. For example, in one embodiment, a filter can be attached to the frame 302 as discussed herein.

In some embodiments, the plug portion 314 can be formed from a cross-linkable polymer 336 positioned inside the frame 302 lumen. The cross-linkable polymer 336 can be injected into the frame 302 lumen to form the plug portion 314, where the frame 302 is formed into a mesh configuration to contain the plug portion 314. In such embodiments, the frame 302 can be anchored to the body passage without a plug portion 314. Then, the cross-linkable polymer 336 can be injected into the frame 302 lumen using a tubular body inside of a catheter, as discussed herein, and adhere to the frame 302, thus preventing migration of the cross-linkable polymer 336. The cross-linkable polymer 336 can be used in embodiments where the frame 302 is configured as shown in Figures 2 and/or 3A-3B. However, the cross-linkable polymer 336 can also be used in embodiments where the frame 302 is not formed into a mesh configuration, but is a solid material, like that shown in Figure 1.

In an additional embodiment, the cross-linkable polymer 336 can be injected into a fillable balloon within the frame 302 lumen to expand the frame 302 from the first configuration to the second configuration. The fillable balloon can be attached to the frame 302 using chemical adhesion, adhesives, sutures, staples, or the like.

In embodiments where the cross-linkable polymer 336 is injected into a balloon, the cross-linkable polymer 336 and the balloon can be made of biodegradable materials such that tissue ingrowth can occur over a period of time both into the balloon itself followed by growth into the cross-linkable polymer, or directly into the cross-linkable polymer 336 if there is no balloon. The balloon and/or the cross-linkable polymer 336 may be mixed with impregnated chemotactic or growth factors, collagen gel, collagen fibrils, mitogenic factors, or other determinates which can alter the reaction of the tissue inside the lumen and improve tissue growth.

In an additional embodiment, the cross-linkable polymer 336 can be formed by a free radical reaction with a polymer starting material where a secondary catalyst is added after the polymer starting material. The cross-linkable polymer 336 may be altered by heating, cooling, or exposure to light which may cause it to solidify and form the plug portion. The cross-linkable polymer 336 may be hardened by means of laser energy.

In one embodiment, the cross-linkable polymer 336 can be a polyphosphazine with active chlorine groups that react with hydrogy groups upon contact with water and with amine groups. Thus polyphosphazine that are protected by air or moisture in the balloon, or are in solution to be injected into the balloon are followed by an aqueous solution. The amine or hydroxyl content of the polymer would depend on the pre-reacted portion of the chlorine on the backbone of the polyphosphazine.

In some embodiments, the cross-linkable polymer 336 is formed from polyisocynates and amines or hydroxyl groups. Use of other materials are also possible.

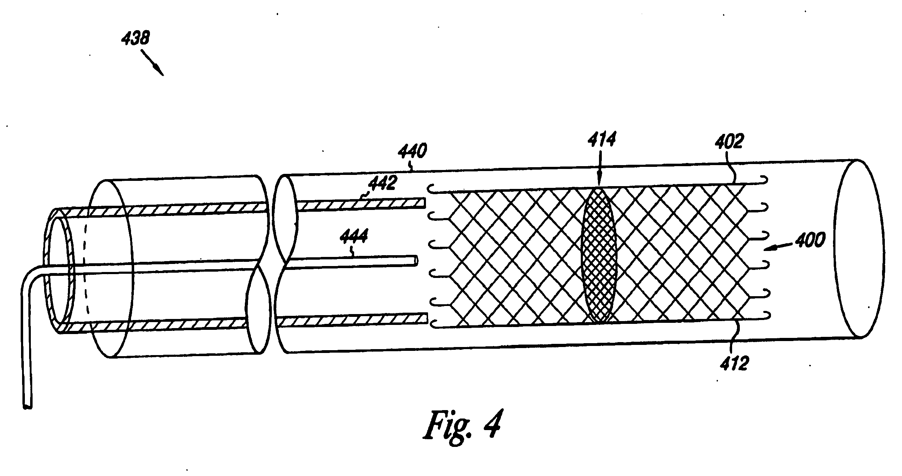

Figure 4 is an illustration of a delivery device 438 according to the present disclosure. As discussed herein, the device 400 can be implanted in several different ways depending on the frame 402 material. In this embodiment, the frame 402 is made of an elastically deformable material that can be formed into the second configuration, and elastically deformed into the first configuration for delivery, as discussed herein. To implant the device 400, the delivery device 438 is a catheter including an exterior tubular body 440 that holds the device 400 in the first configuration, a first interior tubular body 442, and a second interior tubular body 444. The delivery device 438 can be advanced into the interior of the body passage where the first interior tubular body 442 can hold the frame 402 in place inside the body passage while the exterior tubular body 440 is withdrawn. As the exterior tubular body 440 is removed, and the compression force is no longer on the frame 402, the frame 402 can expand from the first configuration to the second configuration and the anchoring members 412 can secure the frame 402 to the inside of the body passage.

In one embodiment, the frame 402 is made of a shape memory alloy that is activated by heat, as discussed herein. The delivery device 438 is advanced into the interior of the body passage where the first interior tubular body 442 can hold the frame in place inside the body passage while the exterior tubular body 440 is withdrawn. To expand the frame 402, a conductive wire can be advanced to contact the frame 402. The conductive wire can then carry a current to the frame 402 to heat the frame 402. Once the frame 402 is heated above its glass transition temperature, the frame 402 will expand from the first configuration, as delivered, to a second configuration to expand the body passage. Once the frame 402 is expanded to the second configuration, the anchoring members 412 can secure the frame 402 to the interior of the body passage.

In one embodiment, the conductive wire can be formed a metal or metal alloy material similar to that used to make the frame, as discussed herein.
In some embodiments, the delivery device 438 can be advanced to the body passage where the first interior tubular body 442 is used to push the frame 402 into the interior of the body passage.

In some embodiments, the second interior tubular body 444 can be placed inside of the first interior tubular body 442, where a cross-linkable polymer is injected through the second interior tubular body 444 into the interior of the frame 402 lumen to form the plug portion 414, as discussed herein.

In the embodiments where the plug portion 414 is formed by injecting a cross-linkable polymer, the frame 402 can be expanded from the first configuration to the second configuration using a catheter equipped with an inflatable balloon. In this embodiment, the frame 402 can be compressed over the balloon in the first configuration, and positioned inside the lumen. The balloon would then be inflated to expand the frame 402 into the second configuration. The balloon can then be deflated, retracted, and the second interior tubular body 444 can be advanced inside the frame 402 lumen to inject the cross-linkable polymer to form the plug portion 414.

As will be appreciated, the exterior tubular body 440, first interior tubular body 442, and second interior tubular body 444 can be formed of a flexible material having sufficient wall strength to resist bending when the delivery device is moved into the body passage. In addition, in some embodiments, the flexible material is also sufficiently rigid to support the pressure of holding the device 400 in the compressed state inside the exterior tubular body 440. In one embodiment, suitable flexible materials include, but are not limited to, polymers such as silicon rubber, polyurethane, and polyethylene. Other suitable materials include Teflon, polyvinyl chloride, Nylon, Dacron, polyetheramide, polyester, polyolefin copolymers, and elastomeric polymers. Use of other materials are also possible.

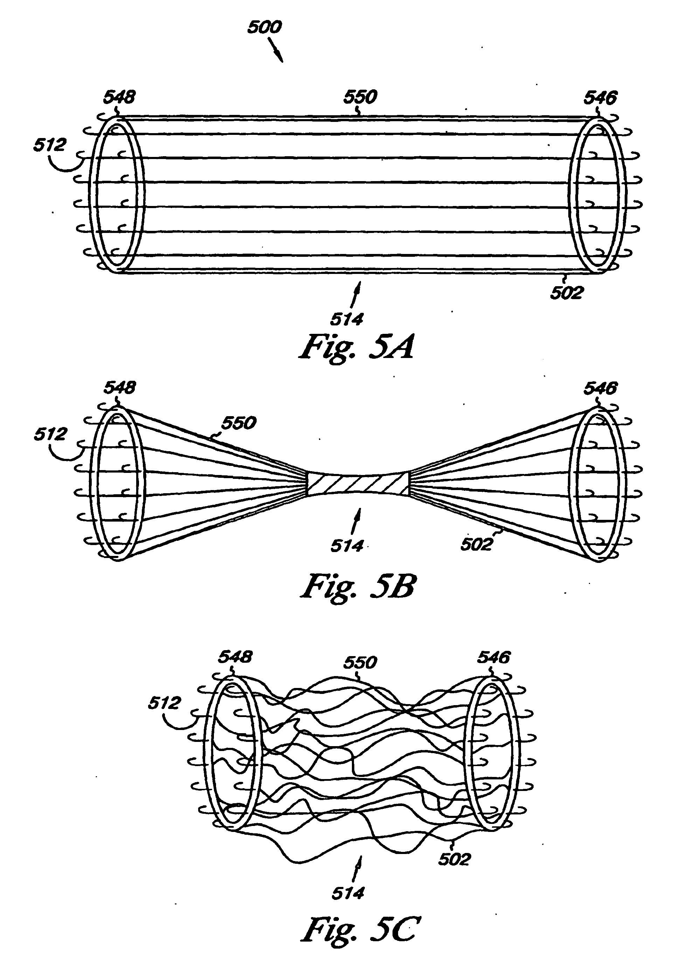

Figures 5A-5C illustrate an additional embodiment of the device 500 according to the present disclosure, where the device 500 in Figure 5A is in the first configuration, and Figures 5B-5C show the device 500 in the second configuration. In this embodiment the device 500 can have a frame 502 including a first expandable ring 546 and a second expandable ring 548 having a plurality of fibers 550 extending between the rings 546, 548. As discussed herein, the frame 502 can be made of a variety of materials. The first and second expandable rings 546, 548 of the frame 502 can be formed of those materials previously discussed.

In this embodiment, the plurality of fibers 550 forms the plug portion 514 to occlude at least a portion of the lumen. In addition, Figures 5A-5C show the plurality of fibers 550 where the fibers are non-woven. In other embodiments, the plurality of fibers 550 can be woven. For example, the plurality of fibers 550 can be woven or knit into a fabric that extends between the first and second expandable rings 546, 548.

Figure 5B illustrates one embodiment where the plurality of fibers 550 can be intertwined to provide the plug portion 514. Figure 5C illustrates an additional embodiment where the plurality of fibers 550 can collapse to provide the plug portion 514. As discussed herein, the plug portion 514 can be formed of a material that will filter the flow of blood through the lumen, or of a material that will stop the flow of blood through the lumen, the plurality of fibers 550 can be constructed of those materials as discussed herein.

Figures 5A-5C also show an embodiment where the first expandable ring 546 and second expandable ring 548 can have anchoring members 512 extending from the frame 502. As discussed herein, the anchoring members 512 can be formed from the frame 502 or formed separately from the frame 502 and coupled to the frame 502. The anchoring members 512 are used to prevent migration of the device 500 once it is implanted in the body passage.

As discussed herein, the plurality of fibers 550 forms the plug portion 514 to occlude at least a portion of the lumen. To accomplish this, first the device 500 is positioned in a body passage through which blood can flow. Next, the device 500 is expanded to secure the device 500 to the body passage. Finally, the body passage is occluded with a plug portion 514 of the device 500 to occlude the flow of blood through the body passage, as discussed herein.

In one embodiment, expanding the device 500 to secure the device 500 to the body passage includes expanding the first expandable ring 546 of the device 500 to secure the first expandable ring 546 to the body passage. A torque is then applied to the second expandable ring 548 of the device 500 to coil a portion of the frame 502 to form the plug portion 514. Next, the second expandable ring 548 of the device 500 is expanded to secure the second expandable ring 548 to the body passage to occlude the flow of blood through the body passage with the plug portion 514 of the device 500. To perform the expansion of the first and second expandable rings 546, 548 separately, the delivery catheter can be equipped with two separate sheaths that can be removed separately, or two inflatable balloons that can be inflated separately, as discussed herein.

In some embodiments, the plug portion 514 can be formed prior to insertion into the body passage by applying a torque to either the first expandable ring or the second expandable ring 546, 548. The device 500, as shown in Figure 5B, can then be placed inside a delivery catheter and positioned in a body passage. After the device 500 is removed from the catheter, the first and second expandable rings 546, 548 can be expanded to secure the device 500 to the body passage. The frame 502 can be expanded by compressing a self-expanding frame 502 from the second configuration to the first configuration, or the like, as discussed herein.

In some embodiments, the device 500 can include a middle ring in the center of the device 500. In various embodiments, the middle ring can be formed of a material that can self-contract to form the plug portion 514 of the device 500. For example, the device 500 can have the first and second expandable rings 546, 548 and the middle ring of approximately equal diameters. However, in this example, after the first and second expandable rings 546, 548 are expanded as discussed herein, the middle ring can contract so that the plurality of fibers 550 are pulled together to form the plug portion 514. In some embodiments the middle ring can be removed after the plug portion 514 is formed. In other embodiments, the middle ring can be made of a biocompatible material and can be left in the body passage.

As discussed herein, Figure 5C shows an additional embodiment of the device 500 where the plug portion 514 is formed by a plurality of fibers 550. In this embodiment, expanding the device 500 to secure the device 500 to the body passage includes expanding the first expandable ring 546 of the device 500 to secure the first expandable ring 546 to the body passage. Next the frame 502 of the device 500 can be compressed to axially collapse a portion of the frame 502 to form the plug portion 514. Then the second expandable ring 548 of the device 500 can be expanded to secure the second expandable ring 548 to the body passage to occlude the flow of blood through the body passage with the plug portion 514 of the device 500. As discussed herein, the delivery catheter can be equipped with multiple separate sheaths that can be removed separately, or multiple balloons that can be inflated separately to expand the first and second expandable rings 546, 548 separately.

As discussed herein, in one embodiment, the device 500 as shown in Figure 5C could be placed in a delivery catheter compressed into the first configuration and positioned in a body passage prior to the expansion of the first and second expandable rings 546, 548. Once the delivery catheter is removed, the first and second expandable rings 546, 548 can expand to the second configuration to secure the frame 502 to the body passage.

In yet another embodiment, the device 500 as shown in Figure 5C can be formed having a first expandable ring 546 and a second expandable ring 548 that are magnetically attracted to each other. In this embodiment, a sheath could be placed between the first and second expandable rings 546, 548 to hold them apart during delivery. Once the frame 502 is positioned inside the lumen, the first expandable ring 546 can be expanded to anchor the first expandable ring 546. The sheath separating the two rings 546, 548 can then be removed. Since the first and second expandable rings 546, 548 are magnetically attracted to each other, once the sheath is removed, the second expandable ring 548 will move towards the first expandable ring 546. The movement of the second expandable ring 548 will allow the plurality of fibers 550 to collapse and form the plug portion 514. The second expandable ring 548 can then be expanded to secure the frame 502 to the lumen, as discussed herein.

In some embodiments, the device 500 as shown in Figure 5C can be formed having a first expandable ring 546 and a second expandable ring 548 that are magnetic. For example, once the frame 502 is positioned inside the lumen, the first expandable ring can be expanded to anchor the first expandable ring 546. To position the second expandable ring 546, a magnet can be supplied between the first expandable ring and the second expandable ring 546, 548. When the magnet is supplied, the second expandable ring 548 can move towards the first expandable ring 546. The movement of the second expandable ring 548 will allow the plurality of fibers 550 to collapse and form the plug portion 514. The magnet can then be removed, and the second expandable ring 548 can be expanded to secure the frame 502 to the lumen, as discussed herein.

By using the plurality of fibers 550 as illustrated in the embodiments shown in Figures 5A-5C, the length of the frame 502 can be adjusted for the particular size of the cardiac defect to be treated. For example, when the plurality of fibers 550 are intertwined as shown in Figure 5B, the length of the frame 502 can be shortened or lengthened depending on how many rotations are applied to either the first or second expandable ring 546, 548 to produce the plug portion 514, as discussed herein. Similarly, the embodiment illustrated in Figure 5C can have an adjustable length depending on how far the first and second expandable rings 546, 548 are compressed together, as discussed herein.

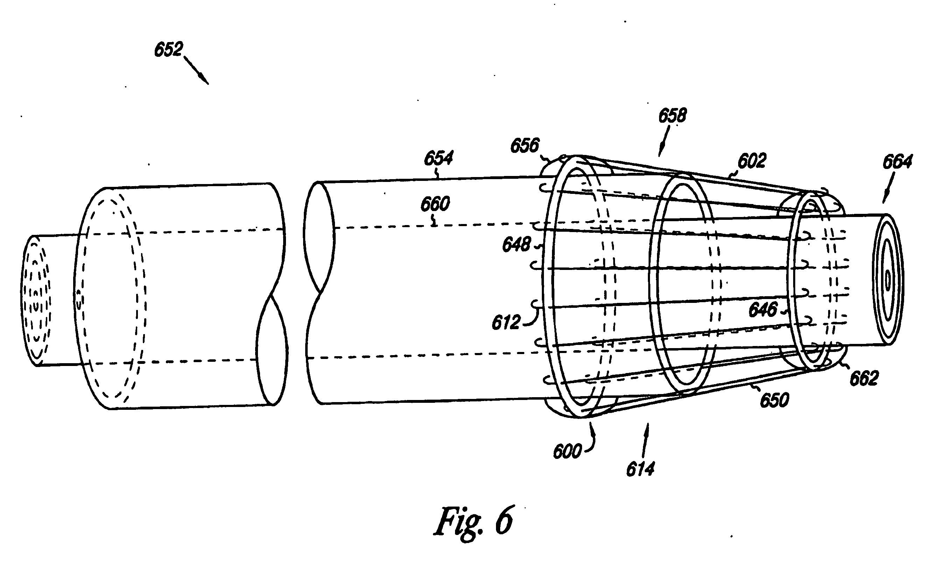

Figure 6 is an illustration of an embodiment of a delivery system 652 according to the present disclosure. The delivery system 652 includes an interior balloon catheter 660 with an inflation lumen with a fluid-tight connection to a first expandable balloon 662 positioned adjacent the interior balloon catheter 660 at the distal end 664 of the interior balloon catheter 660. In addition, the delivery system 652 includes an exterior balloon catheter 654 with an inflation lumen with a fluid-tight connection to a second expandable balloon 656 positioned adjacent the exterior balloon catheter 654 at the distal end 658 of the exterior balloon catheter 654. In addition, the exterior balloon catheter 654 further includes a lumen in which the interior balloon catheter 660 can be placed. The exterior and interior balloon catheters 654, 660 can be configured such that they can move longitudinally and radially relative to each other. In addition, the system 652 can further include a guidewire lumen associated with the interior balloon catheter 660, where the guidewire lumen can be positioned concentrically or eccentrically within the interior balloon catheter 660 lumen.

To implant the device 600, the system 652 is advanced through the body to the body passage opening. Once at the opening, the exterior balloon catheter 654 and interior balloon catheter 660 are moved into the body passage simultaneously. In this embodiment, once the frame 602, such as the frame 602 embodiments described herein, is inside the body passage, the first expandable balloon 662 can be inflated using the inflation lumen. As the first expandable balloon 662 inflates, the first expandable ring 646 can expand from the first configuration, as delivered, to the second configuration. By expanding the first expandable ring 646, the anchoring members 612 can engage the interior tissue of the body passage to fix the position of the first expandable ring 646. Once the first expandable ring 646 is anchored to the interior of the body passage, the first expandable balloon 662 can be deflated and the interior balloon catheter 660 can be retracted so that it is no longer inside the frame 602 lumen. Once the first expandable balloon 662 is removed, a torque can be applied to the exterior balloon catheter 654 to twist the second expandable ring 648, thereby intertwining the plurality of fibers 650 into a coil to form the plug portion 614. Once the plug portion 614 is formed, the second expandable balloon 656 can be inflated to expand the second expandable ring 648 from the first configuration to the second configuration, as discussed herein. By expanding the second expandable ring 648, the anchoring members 612 on the second expandable ring 648 engage the interior of the body passage and fix the second expandable ring 648 in position. Once the second expandable ring 648 is in place, the second expandable balloon 654 can be deflated and the delivery system 652 can be removed from the body.

As discussed herein, the embodiment of the delivery system 652 as illustrated in Figure 6 can also be used with the device as illustrated in Figure 5A and 5C.

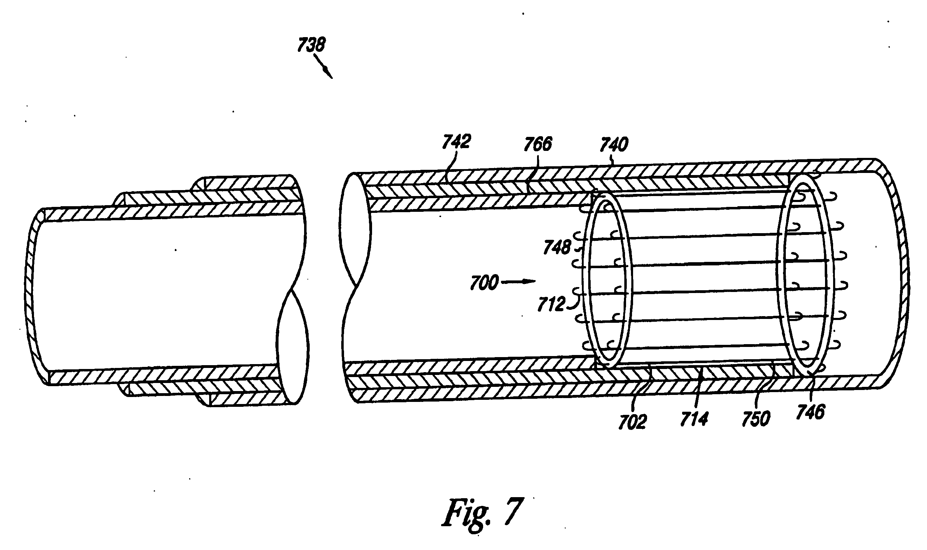

Figure 7 is an illustration of an embodiment of a delivery device 738 according to the present disclosure. The delivery device 738 includes an exterior tubular body 740, a first interior tubular body 742, and an inner tubular body 766. The exterior, first interior, and inner tubular bodies 740, 742, 766 can be configured such that they can move longitudinally relative to each other and/or rotate relative to each other. In one embodiment, the device 700 has a frame 702 that is made from a material that is elastically deformable, as discussed herein. In this embodiment, the first expandable ring 746 is compressed inside of the exterior tubular body 740, and the second expandable ring 748 is compressed inside of the first interior tubular body 742. The first interior tubular body 742 abuts the first expandable ring 746, while the inner tubular body 766 abuts the second expandable ring 748. The delivery device 738 is advanced so that the delivery device 738 is inside the body passage. The exterior tubular body 740 is removed from the first expandable ring 746 while the first interior tubular body 742 holds the first expandable ring 746 in place. Once the exterior tubular body 740 is removed, the first expandable ring 746 can expand to the second configuration and the anchoring members 712 can engage the tissue of the body passage to anchor the first expandable ring 746. Next, a torque can be applied to the second expandable ring 748 by twisting the first interior tubular body 742. The plug portion 714 can be formed when the plurality of fibers 750 is intertwined. Once the plug portion 714 is formed, the first interior tubular body 742 and the exterior tubular body 740 are retracted to release the second expandable ring 748 while the inner tubular body 766 holds the second expandable ring 748 in place. Once the exterior tubular body 740 and first interior tubular body 742 are removed, the second expandable ring 748 can expand to the second configuration and the anchoring members 712 can engage the tissue of the body passage to anchor the second expandable ring 746.

In an additional embodiment, the device 700 is made from a heat activated SMP. In this embodiment, a delivery device 738 equipped with a conductive wire can be used to expand the frame 702 from the first configuration to the second configuration. In this embodiment, the delivery system 738 is advanced so that the delivery system 738 is inside of the body passage. While the first interior tubular body 742 is abutting the frame 702, the exterior tubular body can be retracted so that the first expandable ring 746 is no longer inside of the exterior tubular body 740. At this time, the conductive wire can be advanced to contact the first expandable ring 746, carry a current to the first expandable ring 746, and heat the first expandable ring 746. Once the first expandable ring 746 is heated above its glass transition temperature, the first expandable ring 746 can expand to the second configuration to anchor the first expandable ring 746 inside the body passage. As discussed herein, a torque can be applied to the second expandable ring 748 to intertwine the plurality of fibers 750 to form the plug portion 714. Once the plug portion 714 is formed, the exterior tubular body 740 and the first interior tubular body 742 can be retracted and the conductive wire can be advanced to contact and expand the second expandable ring 748 to secure the second expandable ring 748 to the interior of the body passage, as discussed herein.

In an additional embodiment, the delivery device 738 can be used to collapse the plurality of fibers 750 to form the plug portion 714. In this embodiment, the delivery device 738 is advanced into the interior of the body passage. The exterior tubular body 740.can be retracted to allow the first expandable ring 746 to expand to the second configuration while the first interior tubular body 742 holds the first expandable ring 746 in place inside the body passage. Once the first expandable ring 746 is in the second configuration, the anchoring members 712 can engage the interior tissue of the body passage to fix the first expandable ring 746 to the body passage. The first interior tubular body 742 and exterior tubular body 740 can then be retracted while the inner tubular body 766 holds the second expandable ring 748 in place until the first interior tubular body 742 and exterior tubular body 740 are positioned over the second expandable ring 748. At this time, the exterior tubular body 740, the first interior tubular body 742, and the inner tubular body 766 are pushed towards the first expandable ring 746 simultaneously to collapse the plurality of fibers 750 to form the plug portion 714. Then, while the inner tubular body 766 holds the second expandable ring 748 in place, the exterior tubular body 740 and first interior tubular body 742 can be retracted to allow the second expandable ring 748 to expand to the second configuration. Upon expansion, the anchoring members 712 can engage the tissue of the body passage to fix the second expandable ring 748 in place.

## Claims

1. A medical occlusion device (100, 200, 300, 400, 500, 600, 700), comprising:
a frame (102, 202, 302, 402, 502, 602, 702) defining a lumen and having a first expandable ring (546, 646, 746) and a second expandable ring (548, 648, 748), where the frame (102, 202, 302, 402, 502, 602, 702) expands from a first configuration to a second configuration larger than the first configuration;
an anchoring members(112, 212, 412, 512, 612, 712) extending from the frame (102, 202, 302, 402, 502, 602, 702) to secure the frame (102, 202, 302, 402, 502, 602, 702) while in the second configuration; and
a plurality of fibers (550, 650, 750) extending between the first (546, 646, 746) and second expandable rings (548, 648, 748) to form a plug portion (114, 314, 414, 514, 614, 714) that occludes at least a portion of the lumen.

2. The device (100, 200, 300, 400, 500, 600, 700) of claim 1, where the plurality of fibers (550, 650, 750) can intertwine to provide the plug portion (114, 314, 414, 514, 614, 714).

3. The device (100, 200, 300, 400, 500, 600, 700) of any one of the preceding claims, where the plurality of fibers (550, 650, 750) can collapse to provide the plug portion (114, 314,414, 514, 614, 714).

4. The device (100, 200, 300, 400, 500, 600, 700) of any one of the preceding claims, further including an interior balloon catheter (660) with an inflation lumen with a fluid-tight connection to a first expandable balloon (662) positioned adjacent the interior balloon catheter (660) at a distal end (664) of the interior balloon catheter (660), and an exterior balloon catheter (654) with an inflation lumen with a fluid-tight connection to a second expandable balloon (656) positioned adjacent the exterior balloon catheter (654) at a distal end (658) of the exterior balloon catheter (654), the exterior balloon catheter (654) having a lumen in which the interior balloon catheter (660) can be placed and the exterior (654) and interior balloon catheters (660) being configured so they can move longitudinally and radially relative to each other, and
where the frame (102, 202, 302, 402, 502, 602, 702) is positioned over the first expandable balloon (662) and the second expandable balloon (656) to allow the first expandable balloon (662) to expand the first expandable ring (546, 646, 746) as the first expandable balloon (662) inflates, a torque to be applied to the exterior balloon catheter (654) to twist the second expandable ring (548, 648, 748), thereby intertwining the plurality of fibers (550, 650, 750) into a coil to form the plug portion (114, 314, 414, 514, 614, 714), and to expand the second expandable ring (548, 648, 748) from the first configuration to the second configuration as the second expandable balloon (656) inflates.

5. The device (100, 200, 300, 400, 500, 600, 700) of any one of the preceding claims, further including an exterior tubular body (740), a first interior tubular body (742), and an inner tubular body (766), the exterior (740), first interior (742), and inner tubular (766) bodies being configured to move longitudinally relative to each other and/or rotate relative to each other, the first expandable ring (546, 646, 746) of the frame (102, 202, 302, 402, 502, 602, 702) being compressed inside of the exterior tubular body (740), and the second expandable ring (548, 648, 748) being compressed inside of the first interior tubular body (742), where the first interior tubular body (742) abuts the first expandable ring (546, 646, 746), while the inner tubular body (766) abuts the second expandable ring (548, 648, 748), and where the exterior tubular body (740) can be removed from the first expandable ring (546, 646, 746) to allow the first expandable ring (546, 646, 746) to expand to the second configuration, a torque can be applied to the second expandable ring (548, 648, 748) by twisting the first interior tubular body (742) to form a plug portion (114, 314, 414, 514, 614, 714) with the plurality of fibers (550, 650, 750), and the first interior tubular body (742) and the exterior tubular body (740) can retract to release the second expandable ring (548, 648, 748) to expand to the second configuration.

6. The device (100, 200, 300, 400, 500, 600, 700) of any one of the preceding claims, where the plug portion (114, 314, 414, 514, 614, 714) is a filter (116) coupled to an inside perimeter of the frame (102, 202, 302, 402, 502, 602, 702).

7. The device (100, 200, 300, 400, 500, 600, 700) of any one of the preceding claims, where the frame (102, 202, 302, 402, 502, 602, 702) includes a first end portion (224, 324), a second end portion (226, 326), and a middle portion (222, 322), where the first end portion (224, 324) and second end portion (226, 326) in the second configuration have a larger perimeter than the middle portion (222, 322) in the second configuration.

8. The device (100, 200, 300, 400, 500, 600, 700) of any one of the preceding claims, where the plug portion (114, 314, 414, 514, 614, 714) is a cross-linkable polymer, and is positioned inside the frame (102, 202, 302, 402, 502, 602, 702).

9. The device (100, 200, 300, 400, 500, 600, 700) of any one of the preceding claims, further including a delivery catheter to provide for a delivery system (652).

10. The device (100, 200, 300, 400, 500, 600, 700) of any one of the preceding claims, where the device (100, 200, 300, 400, 500, 600, 700) is formed of a first piece including the anchoring member (112, 212, 412, 512, 612, 712) extending from the frame (102, 202; 302, 402, 502, 602, 702) and a second piece including the plug portion (114, 314, 414, 514, 614, 714), and the first piece is coupled to the second piece.

11. A method of forming a medical occlusion device, comprising:
forming a frame (102, 202, 302, 402, 502, 602, 702) defining a lumen and having a first expandable ring (546, 646, 746), a second expandable ring (548, 648, 748), where the frame (102, 202, 302, 402, 502, 602, 702) can expand from a first configuration to a second configuration larger than the first configuration;
forming an anchoring member (112, 212, 412, 512, 612, 712) extending from the frame (102, 202, 302, 402, 502, 602, 702); and
providing for a plug portion (114, 314, 414, 514, 614, 714) in the lumen of the frame (102, 202, 302, 402, 502, 602, 702) from a plurality of fibers (550, 650, 750) extending between the first (546, 646, 746) and second expandable rings (548, 648, 748), where the plug (114, 314, 414, 514, 614, 714) can occlude at least a portion of the lumen.

12. The method of claim 11, where providing for the plug portion (114, 314, 414, 514,614,714) further includes intertwining the plurality of fibers (550, 650, 750) to form the plug portion (114, 314, 414, 514, 614, 714).

13. The method of claim 11 or 12, where providing for the plug portion (114, 314, 414, 514, 614, 714) further includes collapsing the plurality of fibers (550, 650, 750) to form the plug portion (114, 314, 414, 514, 614, 714).

14. The method of any one of claims 11-13, where providing for the plug portion (114, 314, 414, 514, 614, 714) further includes coupling a filter to an inside perimeter of the frame (102, 202, 302, 402, 502, 602, 702) to form the plug portion (114, 314, 414, 514, 614, 714).

15. The method of any one of claims 11-14, where providing for the plug portion (114, 314, 414, 514, 614, 714) further includes injecting a cross-linkable polymer into the frame (102, 202, 302, 402, 502, 602, 702) to form the plug portion (114, 314, 414, 514, 614, 714).

## Patentansprüche

1. Medizinische Okklusionsvorrichtung (100, 200, 300, 400, 500, 600, 700) mit:
einem Rahmen (102, 202, 302, 402, 502, 602, 702), der ein Lumen definiert und einen ersten expandierbaren Ring (546, 646, 746) und einen zweiten expandierbaren Ring (548, 648, 748) hat, wobei der Rahmen (102, 202, 302, 402, 502, 602, 702) aus einer ersten Konfiguration in eine zweite Konfiguration expandiert, die größer als die erste Konfiguration ist;
einem Verankerungsteil (112, 212, 412, 512, 612, 712), das sich vom Rahmen (102, 202, 302, 402, 502, 602, 702) erstreckt, um den Rahmen (102, 202, 302, 402, 502, 602, 702) zu befestigen, während er sich in der zweiten Konfiguration befindet; und
mehreren Fasern (550, 650, 750), die sich zwischen dem ersten (546, 646, 746) und zweiten expandierbaren Ring (548, 648, 748) erstrecken, um einen Stopfenabschnitt (114, 314, 414, 514, 614, 714) zu bilden, der mindestens einen Abschnitt des Lumens okkludiert.

2. Vorrichtung (100, 200, 300, 400, 500, 600, 700) nach Anspruch 1, wobei sich die mehreren Fasern (550, 650, 750) verschlingen können, um den Stopfenabschnitt (114, 314, 414, 514, 614, 714) vorzusehen.

3. Vorrichtung (100, 200, 300, 400, 500, 600, 700) nach einem der vorstehenden Ansprüche, wobei die mehreren Fasern (550, 650, 750) kollabieren können, um den Stopfenabschnitt (114, 314, 414, 514, 614, 714) vorzusehen.

4. Vorrichtung (100, 200, 300, 400, 500, 600, 700) nach einem der vorstehenden Ansprüche, die ferner aufweist: einen innenseitigen Ballonkatheter (660) mit einem Aufblaslumen mit einer fluiddichten Verbindung zu einem ersten expandierbaren Ballon (662), der benachbart zum innenseitigen Ballonkatheter (660) an einem distalen Ende (664) des innenseitigen Ballonkatheters (660) positioniert ist, und einen außenseitigen Ballonkatheter (654) mit einem Aufblaslumen mit einer fluiddichten Verbindung zu einem zweiten expandierbaren Ballon (656), der benachbart zum außenseitigen Ballonkatheter (654) an einem distalen Ende (658) des außenseitigen Ballonkatheters (654) positioniert ist, wobei der außenseitige Ballonkatheter (654) ein Lumen hat, in dem der innenseitige Ballonkatheter (660) platziert sein kann, und der außenseitige (654) und innenseitige Ballonkatheter (660) so konfiguriert sind, dass sie sich relativ zueinander längs und radial bewegen können, und wobei der Rahmen (102, 202, 302, 402, 502, 602, 702) über dem ersten expandierbaren Ballon (662) und dem zweiten expandierbaren Ballon (656) so positioniert ist, dass der erste expandierbare Ballon (662) den ersten expandierbaren Ring (546, 646, 746) expandieren kann, wenn sich der erste expandierbare Ballon (662) aufbläst, ein Drehmoment am außenseitigen Ballonkatheter (654) ausgeübt werden kann, um den zweiten expandierbaren Ring (548, 648, 748) zu drehen, wodurch die mehreren Fasern (550, 650, 750) zu einer Spule verschlungen werden, um den Stopfenabschnitt (114, 314, 414, 514, 614, 714) zu bilden, und der zweite expandierbare Ring (548, 648, 748) aus der ersten Konfiguration in die zweite Konfiguration expandiert werden kann, wenn sich der zweite expandierbare Ballon (656) aufbläst.

5. Vorrichtung (100, 200, 300, 400, 500, 600, 700) nach einem der vorstehenden Ansprüche, die ferner aufweist: einen außenseitigen Röhrenkörper (740), einen ersten innenseitigen Röhrenkörper (742) und einen Innenröhrenkörper (766), wobei der außenseitige (740), erste innenseitige (742) und Innenröhren- (766) Körper so konfiguriert sind, dass sie sich relativ zueinander längs bewegen und/oder relativ zueinander drehen, der erste expandierbare Ring (546, 646, 746) des Rahmens (102, 202, 302, 402, 502, 602, 702) innerhalb des außenseitigen Röhrenkörpers (740) zusammengedrückt ist und der zweite expandierbare Ring (548, 648, 748) innerhalb des ersten innenseitigen Röhrenkörpers (742) zusammengedrückt ist, wobei der erste innenseitige Röhrenkörper (742) am ersten expandierbaren Ring (546, 646, 746) anliegt, während der Innenröhrenkörper (766) am zweiten expandierbaren Ring (548, 648, 748) anliegt, und wobei der außenseitige Röhrenkörper (740) vom ersten expandierbaren Ring (546, 646, 746) entfernt werden kann, damit der erste expandierbare Ring (546, 646, 746) in die zweite Konfiguration expandieren kann, ein Drehmoment am zweiten expandierbaren Ring (548, 648, 748) durch Drehen des ersten innenseitigen Röhrenkörpers (742) ausgeübt werden kann, um einen Stopfenabschnitt (114, 314, 414, 514, 614, 714) mit den mehreren Fasern (550, 650, 750) zu bilden, und sich der erste innenseitige Röhrenkörper (742) und der außenseitige Röhrenkörper (740) zurückziehen können, um den zweiten expandierbaren Ring (548, 648, 748) freizugeben, um in die zweite Konfiguration zu expandieren.

6. Vorrichtung (100, 200, 300, 400, 500, 600, 700) nach einem der vorstehenden Ansprüche, wobei der Stopfenabschnitt (114, 314, 414, 514, 614, 714) ein Filter (116) ist, das mit einem Innenumfang des Rahmens (102, 202, 302, 402, 502, 602, 702) gekoppelt ist.

7. Vorrichtung (100, 200, 300, 400, 500, 600, 700) nach einem der vorstehenden Ansprüche, wobei der Rahmen (102, 202, 302, 402, 502, 602, 702) einen ersten Endabschnitt (224, 324), einen zweiten Endabschnitt (226, 326) und einen Mittelabschnitt (222, 322) aufweist, wobei der erste Endabschnitt (224, 324) und zweite Endabschnitt (226, 326) in der zweiten Konfiguration einen größeren Umfang als der Mittelabschnitt (222, 322) in der zweiten Konfiguration haben.

8. Vorrichtung (100, 200, 300, 400, 500, 600, 700) nach einem der vorstehenden Ansprüche, wobei der Stopfenabschnitt (114, 314, 414, 514, 614, 714) ein vernetzbares Polymer ist und innerhalb des Rahmens (102, 202, 302, 402, 502, 602, 702) positioniert ist.

9. Vorrichtung (100, 200, 300, 400, 500, 600, 700) nach einem der vorstehenden Ansprüche, ferner mit einem Abgabekatheter, um für ein Abgabesystem (652) zu sorgen.

10. Vorrichtung (100, 200, 300, 400, 500, 600, 700) nach einem der vorstehenden Ansprüche, wobei die Vorrichtung (100, 200, 300, 400, 500, 600, 700) gebildet ist aus einem ersten Stück mit dem Verankerungsteil (112, 212, 412, 512, 612, 712), das sich vom Rahmen (102, 202, 302, 402, 502, 602, 702) erstreckt, und einem zweiten Stück mit dem Stopfenabschnitt (114, 314, 414, 514, 614, 714), und das erste Stück mit dem zweiten Stück gekoppelt ist.

11. Verfahren zur Bildung einer medizinischen Okklusionsvorrichtung, das aufweist:
Bilden eines Rahmens (102, 202, 302, 402, 502, 602, 702), der ein Lumen definiert und einen ersten expandierbaren Ring (546, 646, 746) und einen zweiten expandierbaren Ring (548, 648, 748) hat, wobei der Rahmen (102, 202, 302, 402, 502, 602, 702) aus einer ersten Konfiguration in eine zweite Konfiguration expandieren kann, die größer als die erste Konfiguration ist;
Bilden eines Verankerungsteils (112, 212, 412, 512, 612, 712), das sich vom Rahmen (102, 202, 302, 402, 502, 602, 702) erstreckt; und
Vorsehen eines Stopfenabschnitts (114, 314, 414, 514, 614, 714) im Lumen des Rahmens (102, 202, 302, 402, 502, 602, 702) aus mehreren Fasern (550, 650, 750), die sich zwischen dem ersten (546, 646, 746) und zweiten expandierbaren Ring (548, 648, 748) erstrecken, wobei der Stopfen (114, 314, 414, 514, 614, 714) mindestens einen Abschnitt des Lumens okkludieren kann.

12. Verfahren nach Anspruch 11, wobei das Vorsehen des Stopfenabschnitts (114, 314, 414, 514, 614, 714) ferner aufweist: Verschlingenlassen der mehreren Fasern (550, 650, 750), um den Stopfenabschnitt (114, 314, 414, 514, 614, 714) zu bilden.

13. Verfahren nach Anspruch 11 oder 12, wobei das Vorsehen des Stopfenabschnitts (114, 314, 414, 514, 614, 714) ferner aufweist: Kollabierenlassen der mehreren Fasern (550, 650, 750), um den Stopfenabschnitt (114, 314, 414, 514, 614, 714) zu bilden.

14. Verfahren nach einem der Ansprüche 11-13, wobei das Vorsehen des Stopfenabschnitts (114, 314, 414, 514, 614, 714) ferner aufweist: Koppeln eines Filters mit einem Innenumfang des Rahmens (102, 202, 302, 402, 502, 602, 702), um den Stopfenabschnitt (114, 314, 414, 514, 614, 714) zu bilden.

15. Verfahren nach einem der Ansprüche 11-14, wobei das Vorsehen des Stopfenabschnitts (114, 314, 414, 514, 614, 714) ferner aufweist: Einspritzen eines vemetzbaren Polymers in den Rahmen (102, 202, 302, 402, 502, 602, 702), um den Stopfenabschnitt (114, 314, 414, 514, 614, 714) zu bilden.

## Revendications

1. Dispositif d'occlusion médical (100, 200, 300, 400, 500, 600, 700), comprenant :
une armature (102, 202, 302, 402, 502, 602, 702) définissant une lumière et pourvue d'une première bague expansible (546, 646, 746) et d'une deuxième bague expansible (548, 648, 748), ladite armature (102, 202, 302, 402, 502, 602, 702) étant expansible d'une première conformation à une deuxième conformation plus grande que la première conformation ;
un élément d'ancrage (112, 212, 412, 512, 612, 712) faisant saillie de l'armature (102, 202, 302, 402, 502, 602, 702) pour fixer ladite armature (102, 202, 302, 402, 502, 602, 702) lors de la deuxième conformation ; et
une pluralité de fibres (550, 650, 750) s'étendant entre la première (546, 646, 746) et la deuxième bague expansibles (548, 648, 748) pour former une partie tampon (114, 314, 414, 514, 614, 714) qui obture au moins une partie de la lumière.

2. Dispositif (100, 200, 300, 400, 500, 600, 700) selon la revendication 1, où la pluralité de fibres (550, 650, 750) peuvent être entrelacées pour produire la partie tampon (114, 314, 414, 514, 614, 714).

3. Dispositif (100, 200, 300, 400, 500, 600, 700) selon l'une quelconque des revendications précédentes, où la pluralité de fibres (550, 650, 750) peuvent subir un relâchement pour produire la partie tampon (114, 314, 414, 514, 614, 714).

4. Dispositif (100, 200, 300, 400, 500, 600, 700) selon l'une quelconque des revendications précédentes, comprenant en outre un cathéter-ballon intérieur (660) avec une lumière de gonflement présentant une connexion étanche aux fluides avec un premier ballon expansible (662) adjacent au cathéter-ballon intérieur (660) à une extrémité distale (664) dudit cathéter-ballon intérieur (660), et un cathéter-ballon extérieur (654) avec une lumière de gonflement présentant une connexion étanche aux fluides avec un deuxième ballon expansible (656) adjacent au cathéter-ballon extérieur (654) à une extrémité distale (658) dudit cathéter-ballon extérieur (654), ledit cathéter-ballon extérieur (654) ayant une lumière où le cathéter-ballon intérieur (660) peut être mis en place et les cathéters-ballons extérieur (654) et intérieur (660) étant prévus de manière à pouvoir se déplacer longitudinalement et radialement l'un par rapport à l'autre, et où l'armature (102, 202, 302, 402, 502, 602, 702) est disposée par-dessus le premier ballon expansible (662) et le deuxième ballon expansible (656) pour permettre au premier ballon expansible (662) d'élargir la première bague expansible (546, 646, 746) lors du gonflement du premier ballon expansible (662), à un couple de s'appliquer sur le cathéter-ballon extérieur (654) pour tordre la deuxième bague expansible (548, 648, 748), entrelaçant alors la pluralité de fibres (550, 650, 750) en bobine pour former la partie tampon (114, 314, 414, 514, 614, 714), et pour élargir la deuxième bague expansible (548, 648, 748) de la première conformation à la deuxième conformation lors du gonflement du deuxième ballon expansible (656).

5. Dispositif (100, 200, 300, 400, 500, 600, 700) selon l'une quelconque des revendications précédentes, comprenant en outre un corps tubulaire extérieur (740), un premier corps tubulaire intérieur (742), et un corps tubulaire interne (766), lesdits corps tubulaire extérieur (740), premier corps tubulaire intérieur (742), et corps tubulaire interne (766) étant prévus de manière à pouvoir se déplacer longitudinalement l'un par rapport à l'autre et/ou tourner l'un par rapport à l'autre, la première bague expansible (546, 646, 746) de l'armature (102, 202, 302, 402, 502, 602, 702) étant comprimée à l'intérieur du corps tubulaire extérieur (740), et la deuxième bague expansible (548, 648, 748) étant comprimée à l'intérieur du premier corps tubulaire intérieur (742), où le premier corps tubulaire intérieur (742) est attenant à la première bague expansible (546, 646, 746), alors que le corps tubulaire interne (766) est attenant à la deuxième bague expansible (548, 648, 748), et où le corps tubulaire extérieur (740) peut être retiré de la première bague expansible (546, 646, 746) pour permettre à la première bague expansible (546, 646, 746) de s'élargir vers la deuxième conformation, un couple peut être appliqué sur la deuxième bague expansible (548, 648, 748) par torsion du premier corps tubulaire intérieur (742) pour former une partie tampon (114, 314, 414, 514, 614, 714) avec la pluralité de fibres (550, 650, 750), et le premier corps tubulaire intérieur (742) et le corps tubulaire extérieur (740) peuvent être rétractés pour permettre à la deuxième bague expansible (548, 648, 748) de s'élargir vers la deuxième conformation.

6. Dispositif (100, 200, 300, 400, 500, 600, 700) selon l'une quelconque des revendications précédentes, où la partie tampon (114, 314, 414, 514, 614, 714) est un filtre (116) raccordé à un périmètre intérieur de l'armature (102, 202, 302, 402, 502, 602, 702).

7. Dispositif (100, 200, 300, 400, 500, 600, 700) selon l'une quelconque des revendications précédentes, où l'armature (102, 202, 302, 402, 502, 602, 702) comprend une première partie d'extrémité (224, 324), une deuxième partie d'extrémité (226, 326) et une partie centrale (222, 322), où, dans la deuxième conformation, la première partie d'extrémité (224, 324) et la deuxième partie d'extrémité (226, 326) ont un périmètre supérieur à la partie centrale (222, 322).

8. Dispositif (100, 200, 300, 400, 500, 600, 700) selon l'une quelconque des revendications précédentes, où la partie tampon (114, 314, 414, 514, 614, 714) est en polymère réticulable, et est disposée à l'intérieur de l'armature (102, 202, 302, 402, 502, 602, 702).

9. Dispositif (100, 200, 300, 400, 500, 600, 700) selon l'une quelconque des revendications précédentes, comprenant en outre un cathéter porteur à prévoir pour un système de pose (652).

10. Dispositif (100, 200, 300, 400, 500, 600, 700) selon l'une quelconque des revendications précédentes, où ledit dispositif (100, 200, 300, 400, 500, 600, 700) se compose d'une première pièce comprenant l'élément d'ancrage (112, 212, 412, 512, 612, 712) faisant saillie de l'armature (102, 202, 302, 402, 502, 602, 702) et une deuxième pièce comprenant la partie tampon (114, 314, 414, 514, 614, 714), et où la première pièce est accouplée à la deuxième pièce.

11. Procédé permettant de former un dispositif d'occlusion médical, comportant les étapes suivantes :
formation d'une armature (102, 202, 302, 402, 502, 602, 702) définissant une lumière et pourvue d'une première bague expansible (546, 646, 746) et d'une deuxième bague expansible (548, 648, 748), où l'armature (102, 202, 302, 402, 502, 602, 702) est expansible d'une première conformation à une deuxième conformation plus grande que la première conformation ;
formation d'un élément d'ancrage (112, 212, 412, 512, 612, 712) faisant saillie de l'armature (102, 202, 302, 402, 502, 602, 702) ; et
réalisation d'une partie tampon (114, 314, 414, 514, 614, 714) dans la lumière de l'armature (102, 202, 302, 402, 502, 602, 702) à partir d'une pluralité de fibres (550, 650, 750) s'étendant entre la première (546, 646, 746) et la deuxième bague expansibles (548, 648, 748), le tampon (114, 314, 414, 514, 614, 714) pouvant obturer au moins une partie de la lumière.

12. Procédé selon la revendication 11, où la réalisation de la partie tampon (114, 314, 414, 514, 614, 714) comprend en outre un entrelacement de la pluralité de fibres (550, 650, 750) pour former la partie tampon (114, 314, 414, 514, 614, 714).

13. Procédé selon la revendication 11 ou 12, où la réalisation de la partie tampon (114, 314, 414, 514, 614, 714) comprend en outre un relâchement de la pluralité de fibres (550, 650, 750) pour former la partie tampon (114, 314, 414, 514, 614, 714).

14. Procédé selon l'une quelconque des revendications 11 à 13, où la réalisation de la partie tampon (114, 314, 414, 514, 614, 714) comprend en outre le raccordement d'un filtre à un périmètre intérieur de l'armature (102, 202, 302, 402, 502, 602, 702) pour former la partie tampon (114, 314, 414, 514, 614, 714).

15. Procédé selon l'une quelconque des revendications 11 à 14, où la réalisation de la partie tampon (114, 314, 414, 514, 614, 714) comprend en outre l'injection d'un polymère réticulable dans l'armature (102, 202, 302, 402, 502, 602, 702) pour former la partie tampon (114, 314, 414, 514, 614, 714).
